# EUROPEAN PATENT SPECIFICATION

(11) **EP 2 987 862 B1**
(45) Date of publication and mention of the grant of the patent: **08.02.2017**
(21) Application number: 14382320.1
(22) Date of filing: 21.08.2014
(51) Int. Cl.: C12P 7/62, C12R 1/01

(54) **Caulobacter segnis strain and its use for the production of polyhydroxyalkanoates**
Caulobacter segnis-Stamm und dessen Verwendung zur Herstellung von Polyhydroxyalkanoaten
La souche de Caulobacter segnis et son utilisation pour la production de polyhydroxyalkanoates

(43) Date of publication of application: 24.02.2016
(73) Proprietor: Biopolis, S.L., 46980 Paterna, Valencia (ES)
(72) Inventor: Ramón Vidal, Daniel, 46183 La Eliana, Valencia (ES); Tortajada Serra, Marta, 45015 Valencia (ES); Rojas Martínez, Antonia María, 46015 Valencia (ES); Segarra Manzano, Silvia, 46011 Valencia (ES); Bustamante Jaramillo, Daniel Arturo, 46003 Valencia (ES); Prieto Jiménez, Mª Auxiliadora, 28040 Madrid (ES); del Cerro Sánchez, Carlos, 41807 Sevilla (ES); Echevarría Gutiérrez, Francisco Javier, 33429 Siero, Asturias (ES); Barbarie, Philippe Jérôme, 33520 Nava, Asturias (ES); Iglesias Barcia, José Ramón, 33199 Siero, Principado de Asturias (ES)
(74) Representative: Pons

(56) References cited:
- WO-A1-2011/112154
- US-A- 6 117 658
- QINGSHENG QI ET AL: "Polyhydroxybutyrate biosynthesis in Caulobacter crescentus: molecular characterization of the polyhydroxybutyrate synthase", MICROBIOLOGY, vol. 147, 1 January 2001 (2001-01-01), pages 3353-3358, XP055073711, ISSN: 0002-5029

## Description

### FIELD OF THE INVENTION

The present invention relates to a strain of the species *Caulobacter segnis* with deposit number DSM29236, which has the capacity to produce polyhydroxyalkanoates (PHA) from lactose. Therefore, the present invention falls within the field of industrial biotechnology, in particular in the production of biopolymers.

### STATE OF THE ART

Polyhydroxyalkanoates (PHA) are polyesters of microbial origin that are currently marketed as bioplastics. These polymers are thermoplastics and behave similarly to polyolefin, with an advantage over the latter in that they are biodegradable, biocompatible and of renewable origin. Moreover, they demonstrate low oxygen and water permeability levels and can, therefore, be used in packaging. Also their derivatives, obtained by hydrolysis, are optically active molecules, which can be used as high-added-value precursors for the synthesis of compounds of pharmaceutical interest. Depending on the length of the side chain, PHA exhibit higher crystallinity (short-chain PHA, polypropylene analogues) or behave as elastomers (medium-chain PHA, more similar to polyethylene). Their utility has been demonstrated in laminates, packaging, molded articles, elastomers, fibres, adhesives, paints, synthetic paper and foams, as well as biomedical applications such as sutures, implants, and drug delivery systems.

These products are more expensive than their chemical counterparts, fetching around 6-15 €/kg depending on the raw material used. Between 30% and 40% of the processing costs correspond to the cost of the raw material. The use of renewable raw materials is fundamental to obtain profitable and environmentally friendly processes; the former may be waste material from other industrial processes.

Whey is generated in cheese factories on separating the milk curd, and is one of the most polluting materials generated by the food industry due to its high organic and mineral content. It is a product rich in protein, fat and lactose. Studies have assessed the recovery of whey as a source of lactic acid, xanthan gum, lactitol and lactulose. In this respect, it is interesting to consider the production of bioplastics and derivatives thereof from whey.

Some bacteria are able to use the lactose in whey to synthesize PHA. The prior art reports different types of PHA produced from whey or lactose by Gram-negative bacteria (*Methylobacterium* sp. *Pseudomonas* sp. *Thermus thermophilus*) and Gram-positive bacteria, some lactic acid bacteria (genera *Lactobacillus* and *Lactococcus*) and *Bacillus megaterium*. Within the *Caulobacter* genus, the species *C*. *crescentum* has been described as producing PHA from glucose as carbon source (Qi & Rehm, 2001, Microbiology 147: 3353).

The patent US6117658A relates to the production of PHA in a culture medium containing whey and the use of recombinant biosynthetic pathways. Patent application WO2011112154A1 describes a process to produce PHA by culturing PHA-producing microorganisms using lactose as a carbon source.

Therefore, given the state of the art, there is a need to provide useful microorganisms that can be used to industrially produce PHA at higher yields than the PHA productions described in the prior art.

### DETAILED DESCRIPTION OF THE INVENTION

The inventors of the present invention have identified a strain of the species *Caulobacter segnis* which can, surprisingly, produce polyhydroxyalkanoates (PHA) from lactose as a carbon source at higher yields than other PHA-producing microorganisms. This strain has been deposited at the German Collection of Microorganisms and Cell Cultures under deposit number DSM29236.

Based on this strain, the inventors have developed a number of inventive aspects which will be described below.

### Strain of the invention and its uses

One aspect of the invention relates to a strain of the species *Caulobacter segnis* deposited at the German Collection of Microorganisms and Cell Cultures under deposit number DSM29236. Henceforth, this strain will be called "strain of the invention" or "microorganism of the invention."

The strain of the invention was deposited according to the Budapest Treaty on the 7 August 2014 at the German Collection of Microorganisms and Cell Cultures - Leibniz Institute DSMZ (Leibniz-Institut DSMZ-Deutsche Sammlung von Mikro- Organismen und Zellkulturen GmbH) and was allocated deposit number DSM29236. The address of the depositary institution is Inhoffenstraße 7B, 38124, Braunschweig, Germany.

The strain of the invention belongs to the species *Caulobacter segnis,* a gram-negative rod- or vibroid-shaped or fusiform bacterium, which commonly produces a stalk. The strain of the invention belongs to the Kingdom Bacteria; Phylum *Proteobacteria*; Class *Alphaproteobacteria*; Order *Caulobacterales*; Family *Caulobacteraceae;* Gender *Caulobacter.*

The strain of the invention may form part of a bacterial population or form an essentially pure culture. Thus, a bacterial population is a set of microorganism cells where at least one of these organisms is the bacterial strain with deposit number DSM29236, in any phase of its development and at any growth stage, exponential or stationary, regardless of its morphology. Furthermore, in the present invention, the term "essentially pure culture" refers to a bacterial culture wherein at least 95% of the cells it comprises correspond to the bacterial strain with deposit number DSM29236. In all cases, the culture medium must contain the necessary components (e.g., nutrients) and favourable pH, temperature and humidity conditions for the survival and growth of the strain of the invention. Examples of culture medium components include, but are not limited to, an organic energy source (carbohydrates, proteins, polysaccharides, fats, organic acids, etc.), inorganic energy source (ammonium, nitrites, sulphur, etc.), trace elements, vitamins, growth factors, antifungals, etc. Examples of culture media that can be used to maintain the growth of the strain of the invention include, but are not limited to, Luria Bertani medium and Medium 1 as defined by DSMZ.

Furthermore, the bacterial culture can be solid, liquid, lyophilized, etc. The culture media in liquid form are usually called broths because they contain nutrients dissolved in water (for example, nutrient broth). This format allows for suspensions with a high number of microorganisms. The culture media in solid form are usually prepared from a liquid medium to which solidifying agents are added, such as agar, gelatine or silica gel (e.g., nutrient agar), and are often used to isolate and maintain microorganisms in the laboratory. Alternatively, the culture medium comprising the strain of the invention and the other components can also be lyophilized in order to reduce culture volume and weight, enabling its transport without the need for cold chain. This format allows storage of the culture for long time periods, even up to 5 years, in any situation, maintaining its characteristics/properties. Thus, in a particular embodiment, the strain of the invention is characterized in that it is in liquid, fresh paste, active dry or instant dry form.

The strain of the invention is characterized by having the ability to produce PHA from lactose.

In the present invention the term "ability to produce polyhydroxyalkanoates from lactose" refers to the ability of the microorganism of the invention to break down lactose into glucose and galactose to use these compounds as a carbon source to produce PHA. Therefore, another aspect of the present invention, relates to the use of the strain of the invention to produce PHA from a solution comprising glucose, galactose and/or lactose.

Polyhydroxyalkanoates or PHAs are polymers hydroxyalkanoic acids that are intracellularly synthesized by some microorganisms as a source of carbon and energy which, once extracted from the cell, have similar physical properties to petroleum-based plastics. PHAs are fully biodegradable polymers (they can be produced by many microorganisms inhabiting soil, seas, lakes or waste water), they are produced from renewable carbon sources, and are biocompatible (do not have any toxic effects). The polymerization of hydroxyalkanoic acids, by intracellular enzymes, takes place by the condensation of the carboxyl group of a monomer (hydroxyalkanoic acid) with the next hydroxyl group, forming an ester bond (hence they are also known as biopolyesters). They accumulate as liquid polymers, in the form of mobile and amorphous granules which are located in microbial cytoplasm surrounded by a monolayer of phospholipids containing enzymes, polymerases and depolymerases. Examples of PHA include, but are not limited to, 3-hydroxy-propionic acid (3HP), 3-hydroxy-butyric acid (3HB), 3-hydroxy valeric acid (3HV), 3-hydroxy-hexanoic acid (3HHx), 3-hydroxy-heptanoic acid (3HHp), 3-hydroxy-octanoic acid (3HO), 3-hydroxy-nonanoic acid (3HN), 3-hydroxy-decanoic acid (3HUD), 3-hydroxy-undecanoic acid (3HDD), 3-hydroxy-tetradecanoic acid (3HTD), 3-hydroxy-hexadecanoic acid (3HHxD), 4-Hydroxy-butyric acid (4HB), 4-Hydroxy-valeric acid (4HV), 4-Hydroxy-hexanoic acid (4HHx), 4-hydroxy-heptanoic acid (4HHp), 4-Hydroxy-octanoic acid (4HO), 4-Hydroxy-decanoic acid (4HD), 5-hydroxy-valeric acid (5HV), 5-hydroxy-hexanoic acid (5HHx), 6-hydroxy-dodecanoic acid (6HDD). In a particular embodiment, the PHA is poly-3-hydroxybutyrate (PHB).

Glucose, galactose and lactose are sugars used by the strain of the invention as a carbon source from which PHA is synthesized. The raw material from which these sugars can be obtained is widely known in the prior art. Preferably, these sugars will be in a solution that is, diluted in a liquid medium, mainly water. Glucose sources include, but are not limited to, molasses, hydrolyzed lignocellulosic material and starch hydrolysates. Sources of galactose and lactose include, for example, mammals' milk, including cow's milk and its derivatives, such as milk whey or permeate. Thus, in a particular embodiment, the solution comprising glucose, galactose and/or lactose is milk whey, or permeate resulting from the production of concentrated proteins from milk whey.

In the present invention the term "whey" refers to the liquid fraction of the milk that is separated from the curd during cheese production, which comprises, in general, 94% water, 4.5% lactose, 1% protein and 0.5% salts.

In the present invention the term "permeate" refers to the product obtained from the extraction of protein and fat from milk by ultrafiltration of milk, partly skimmed milk (semi-skimmed) or skimmed milk (skimmed).

As the skilled person knows, the use of whey as a base for bacterial cultures requires the sterilization thereof. In the context of the present invention, any of the sterilization techniques of culture media existing in the prior art can be used to sterilize the whey or permeate. Examples of sterilization techniques include, but are not limited to, filtration, radiation and heat. For filtration, whey or permeate is passed through filters of a certain pore size that retain the substances/particles/microorganisms exceeding the pore size. Thus, in a particular embodiment, the filter pore size is 25 µm, 8 µm, 0.45 µm and 0.22 µm. In the case of using heat to sterilize the whey or permeate, this sterilization may be accomplished by using an autoclave, for example at 121 °C and pressure of 0.5 atm.

In a particular embodiment, the production of PHA by the strain of the invention is performed in a culture medium under aerobic conditions, wherein:
- The culture medium comprises a source of phosphorus, a source of inorganic nitrogen and a solution comprising glucose, galactose and/or lactose, and
- The aerobic conditions comprise oxygen saturation levels of at least 10% preferably, at least 20% or at least 30%.

All particular embodiments of the culture medium used in this aspect of the invention will be explained below in the inventive aspect concerning the PHA production method.

### PHA production method

As explained at the beginning of the present description, the strain of the invention has the ability to produce PHA from glucose, galactose and/or lactose as a carbon source. Therefore, the inventors have developed a PHA production method using the strain of the invention.

Thus another aspect the invention relates to a PHA production method, hereinafter "method of the invention", which comprises cultivating the strain of the invention in a culture medium under aerobic conditions, wherein:
- The culture medium comprises a source of phosphorus, a source of inorganic nitrogen and a solution comprising glucose, galactose and/or lactose, and
- The aerobic conditions comprising oxygen saturation levels of at least 10%, preferably, at least 20% or at least 30%.

PHA production comprises the culture of the strain of the invention in a culture medium suitable for PHA production. Said culture medium comprises a source of phosphorus, a source of inorganic nitrogen and a solution comprising a carbon source selected from the group consisting of glucose, galactose, lactose and combinations thereof.

The phosphorus source can be from any compound containing phosphorus which can be assimilated by a living organism. It is usually required to be in the form of phosphates, either organic (hydrolysed by extracellular or periplasmic phosphatase) or inorganic. Phosphorus is mainly used to synthesize nucleic acids and phospholipids, but is also present in coenzymes and proteins. Compounds which can be used as a source of phosphorus in the context of the present invention include, but are not limited to, sodium and potassium phosphates, for example Na₂HPO₄ and KH₂PO₄.

Nitrogen is present in the cell in a reduced state, wherein the radical -NH₂ forms part of the amino acid and nitrogen bases. Many heterotrophic bacteria assimilate these elements in oxidized inorganic combined form such as NO₃⁻, through the sequential action of assimilatory nitrate reductases and nitrite reductases. Some may use the reduced form of inorganic nitrogen, such as ammonium (NH₄⁺). The majority of bacteria able to use ammonium as a sole nitrogen source can also use nitrates. Compounds which may be employed as an inorganic nitrogen source include, but are not limited to, KNO₃, NH₄Cl, (NH₄)₂SO₄, and peptones. Combined nitrogen and phosphorus sources such as (NH₄)₂HPO₄ can also be employed.

In addition to the nitrogen and phosphorus source, the culture medium also contains a carbon source selected from the group consisting of glucose, galactose, lactose and combinations thereof. As explained above, the raw material from which these sugars can be extracted is widely known in the prior art. Preferably, these sugars will be in solution that is, diluted in a liquid medium, mainly water. Glucose sources include, but are not limited to, molasses, hydrolysed lignocellulosic biomass, starch hydrolyzate, etc. Galactose and lactose sources include, but are not limited to, mammals' milk, including cow's milk and its derivatives, such as milk or whey permeate. In a particular embodiment, the solution comprising lactose, galactose and/or glucose is whey or permeate, resulting from the production of concentrated proteins from whey. The terms "whey" and "permeate" have already been defined in previous paragraphs. Additionally, the culture medium may comprise other components such as trace elements (cobalt, zinc, iron, magnesium, manganese, copper, molybdenum, boron, etc.), growth factors, fungal growth inhibitory (antifungal) compounds, vitamins, etc.

The concentration of the components forming the culture medium may vary widely. For example, glucose or galactose concentration may vary between 2 and 20 g/L, between 4 and 16 g /L, preferably 8 g/L or 4 g/L. Lactose concentration may vary, for example between 2 and 16 g/L, between 4 and 12 g/L, preferably, 8 g/L. However, in a particular embodiment, the culture medium comprises between 2 and 20 g/L glucose, preferably, 4 g/L, between 2 and 20 g/L galactose, preferably 4 g/L, and/or between 4 and 16 g/L of lactose, preferably, 8 g/L.

In another particular embodiment, the culture medium comprises:
(a) between 1.5 and 5.5 g/L of a phosphorus source, preferably, between 2.5 and 4.5 g/L, more preferably 3.5 g/L and/or
(b) between 0.05 and 0.3 g/L of an inorganic nitrogen source, preferably between 0.1 and 0.2 g/L.

As the skilled person knows, these concentrations refer to the final concentration of the compound in the culture medium in which the strain of the invention is cultured. The skilled person in the art routinely performs the calculations required to reach the mentioned final concentrations of the said compounds in the culture medium. Other conditions to consider when culturing the strain of the invention to produce PHA include, but are not limited to, pH, humidity, temperature, culture time, etc. In a particular embodiment, the strain is cultured at a pH of between 7.5 and 6.5. In another particular embodiment, the strain is cultured at a temperature of between 28 and 32 °C. In another particular embodiment, the culture time is between 18 and 48 hours.

Another feature of the method of the invention comprises culturing the strain of the invention under aerobic conditions. In the present invention, the term "aerobic conditions" or "oxidative conditions" refer to cultivation of the strain of the invention in the presence of oxygen, specifically, at an oxygen saturation of at least 10%, preferably, 20% or 30 %. In the present description the term "oxygen saturation" refers to the amount of oxygen present in the medium capable of combining with, or be used by the cells.

The strain can be cultured following different culture protocols, for example, continuous culture, batch culture, continuously fed (also called semi-batch, fed-batch) culture, etc.

Continuous culture is a balanced culture maintained indefinitely with an open flow system consisting of: a growth chamber at constant volume supplied by nutrients, from which toxic waste products are removed or separated (by an overflow device). Once the system reaches equilibrium, the number of cells and the concentration of nutrients in the chamber remain constant, at which point it is called a steady state system, with cells growing exponentially.

The batch culture is a type of culture in which the microorganism grows from a limited amount of medium until an essential nutrient is used up or toxic products accumulate to levels which inhibit growth.

Fed batch culture is a batch culture to which fresh medium is added continuously or sequentially without removing the culture that has already grown. This technique is defined as a batch culture which is continuously being fed with a fresh nutrient medium or any of its components.

Once the strain of the invention has been cultured under the above-described conditions, the biomass produced from the growth of the strain of the invention, can be harvested and PHA extracted. Thus, in another particular embodiment, the method of the invention further comprises harvesting the biomass obtained and extracting the PHA produced. In a particular embodiment, the extracted PHA is poly-3-hydroxybutyrate.

Biomass can be harvested by multiple methods, such as filtration, centrifugation, etc.

The most frequently method used to extract PHA from microbial biomass is with chlorinated hydrocarbons using the reflux technique, particularly with chloroform. The resulting PHA solution is filtered to remove cell debris then concentrated and the PHA precipitated in methanol or ethanol. With this technique, the low molecular weight lipids remain in solution and do not interfere with determination. The use of chlorinated solvents is more common for the extraction of short-chain PHA such as P3HB, whereas medium-chain PHAs are soluble in a broader range of solvents. Because large-scale use of the abovementioned solvents can be expensive, it is also possible to use other extraction processes based on ethylene and propylene carbonate, and methodologies based on the release polymer cells by rupturing them with solutions of sodium hypochlorite, acids or bases. In a particular embodiment, the PHA produced is extracted with an organic solvent. Examples of organic solvent include, but are not limited to, chloroform, dichloromethane, acetone, methanol, and mixtures thereof.

After PHA has been removed it can be quantified and physicochemically analyzed. One possible method to quantify PHA is gravimetric, that is, using chloroform as the solvent to extract the polymer from the freeze-dried biomass, and subsequently precipitating it with diethyl ether and acetone, and finally drying and weighing. Another gravimetric quantification method is based on the fact that, under controlled time and temperature conditions, the cell matter (except the granules of P3HB) dissolves in an alkaline solution of sodium hypochlorite so that the bioplastic is released and recovered by centrifugation, and subsequently dried and weighed. Furthermore, the spectrophotometric technique to quantify P3HB is based on the conversion of the polymer to crotonic acid using concentrated sulphuric acid and applying heat. The crotonic acid produced is quantified by UV spectrophotometry reading absorbance at 230 nm, and correlating the values with a standard curve of the acid. It is also possible to use gas chromatography (GC). The most widely used method involves subjecting the lyophilized cells to an acid or alkaline methanolysis. With this process, metal esters are generated with the monomers composing the PHA and then analyzed by GC with a flame ionization detector. Using GC coupled to a mass detector, this technique not only works for quantifying PHA but can also determine their monomer composition. PHA can also be determined in intact cells using dimensional fluorescence spectroscopy and flow cytometry. In this procedure, cells stained with Nile blue exhibit maximum fluorescence between 570 and 605 nm, when excited between 540 and 560 nm, and there is a good correlation between fluorescence intensity and PHA concentration. Another quantification method is ion chromatography based on the conversion of PHA monomers to alkanoic acid via acid propanolysis followed by alkaline hydrolysis with Ca(OH)2, or acid hydrolysis with concentrated H2SO4. An enzymatic quantification method can be used, involving oxidation of 3HB and reoxidation of NADH produced from NAD, leading to formazan production, which is read spectrophotometrically at 492 nm.

Throughout the description and claims the word "comprise" and its variants are not intended to exclude other technical features, additives, components or steps. To experts in the art, other objects, advantages and features of the invention will become apparent from the description and the practical part of the invention. The following examples and figures are provided by way of illustration, and are not intended to be limiting of the present invention.

### BRIEF DESCRIPTION OF THE FIGURES

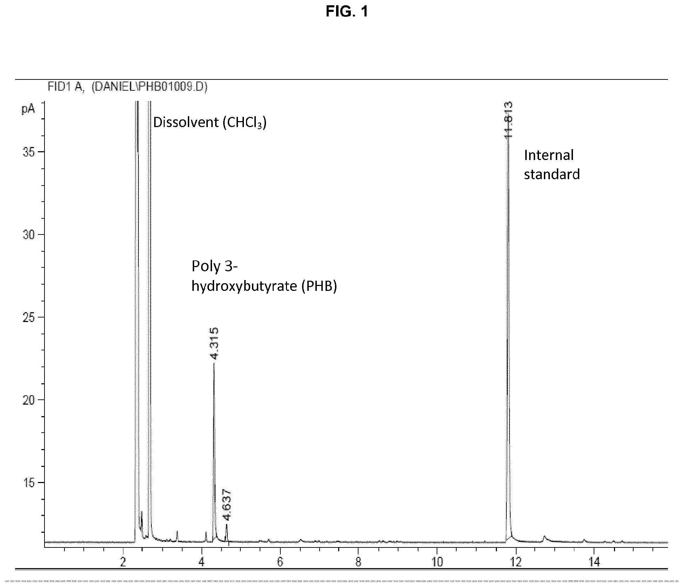

Figure 1 Chromatogram of PHA. The analysis is performed by GC-FID (gas chromatography-flame ionization detector) showing PHB monomers in the *C*. *segnis* DSMZ29236 culture.

### EXAMPLES

Below we illustrate the invention through the experiments performed by the inventors, which demonstrate PHA production by the microorganism of the invention.

### Example 1. Production of polyhydroxyalkanoates by C. segnis DSM29236.

### I. Selection of the C. segnis strain DSM29236.

### a) Identification in silico of PHA-producing microorganisms from lactose.

GRAS bacterial strains capable of growing on lactose and producing PHA were listed from the international depositary authorities. Firstly, a thorough literature study was made in this field and the first six strains were selected and cultivation techniques prepared.

Meanwhile, we conducted a search of sequenced genomes and proteomes of bacteria not described in the literature as PHA producers but which seemed good candidates due to having the orthologous genes required to perform the functions of lactose assimilation and PHA production. Specifically we sought the orthologous genes shown in Table 1 by BLAST (database homology search program) and selected homologies with an "e-value" close to zero (most similar to the sequence model). Thus 42 potential PHA-producing strains from lactose were identified. We discarded from the list all those strains described as pathogenic or those requiring growth conditions difficult to reproduce on an industrial scale due to special requirements, such as vitamins or temperature.

**Table 1 Genes used for searching in silico potential PHA-producing strains from whey.**

| **Gene** | **Organism** |
|---|---|
| β- galactosidase (lacZ) | *Escherichia coli* K12 |
| PHA polymerase Type I | *Ralstonia eutropha* |
| PHA polymerase Type II | *Pseudomonas putida* |
| PHA polymerase Type II | *Pseudomonas putida* |
| PHA polymerase Type III | *Allochromatium vinosum* |
| PHA polymerase Type IV | *Bacillus megaterium* |

### b) Characterization of the whey.

Firstly, it was determined the whey and permeate composition. The term *whey* refers to the liquid fraction of the milk that is separated from the curd during cheese production, whereas *permeate* is produced when protein is concentrated from the whey. We analyzed lactose content, lactic acid, protein and pH. The results are shown in Table 2.

**Table 2. Characterization of whey and permeate to be employed in the formulation of culture media for PHA production.**

| | **Whey** | **Permeate** |
|---|---|---|
| [lactose] g/L | 38 | 56 |
| [lactic acid] g/L | 0.8 | 0.1 |
| [protein] | 6.7 | 3.2 |
| pH | 6.6 | 6.2 |

The content of lactose and lactic acid was determined by HPLC liquid chromatography using a Rezex ROA Organic Acid column, with H₂SO₄ at 2.5 mM and 0.5 mL/minute flow. A differential refractive index detector was used. For subsequent analysis of lactose we used the Colorimetric Kit Megazyme K-LACGAR Lactose and D-Galactose (Rapid). Protein was measured with a ThermoFisher commercial kit (Pierce BCA Protein Assay Kit) using the BCA method.

In order to use the whey as a base for bacterial cultures it must first be sterilized. Two methods were followed: by filtering or by heating in an autoclave. In the former, the whey was passed through several filters (25 µm, 8 µm, 1 µm, 0.45 µm and 0.22 µm) to remove suspended particles and microorganisms; in the latter, sterilization was performed at 121 °C at 0.5 atm in the autoclave.

### c) Evaluation of PHA production from lactose.

Some of the selected bacteria were too small to be examined under light microscopy and distinguish the presence of PHA granules. Therefore it was necessary to develop a method whereby PHA-producing bacteria could be quickly distinguished from non producing bacteria.

The protocol developed is based on staining fatty acids (PHA) with Nile red, the fluorescence increases in correlation with an increase in the amount of accumulated polymer. This method compares the fluorescence of Nile red-stained cells in an environment that does not produce PHA with the fluorescence in a medium that promotes PHA production. *Pseudomonas putida* KT2442 was used as a positive control (good PHA producer) and *Escherichia coli* αDH5 as a negative control (non PHA-producing strain). The non-production conditions were with LB medium whereas PHA accumulation conditions were with M63 medium. This medium is composed of: (NH₄)₂SO₄ 0,2 g/L, MgSO₄·7H₂O 0,25 g/L, KH₂PO₄ 13,6 g/L, FeSO₄.7H₂O 5 mg/L, trace elements 1 mL/L (FeSO₄·7H₂O 2,78 g/L, CaCl₂·H2O 1,47 g/L, ZnSO₄·7H₂O 0,29 g/L, MnCl₂·4H₂O 1,98 g/L, CoSO₄·7H₂O 2,81 g/L, CuCl₂·2H₂O 0,17 g/L); lactose 12 g/L or glucose 20 g/L.

The fluorescence spectrometer used was Hitachi F7000 con λ_{excitation} = 545 nm and λₑₘᵢₛₛᵢₒₙ = 598 nm. Assays were performed in multiwell plates with a volume of 300 µL. To each well 2 µL of a solution of 0.1 g/L of Nile red was added and fluorescence was assessed after 15 min incubation and compared with the reading taken without the dye.

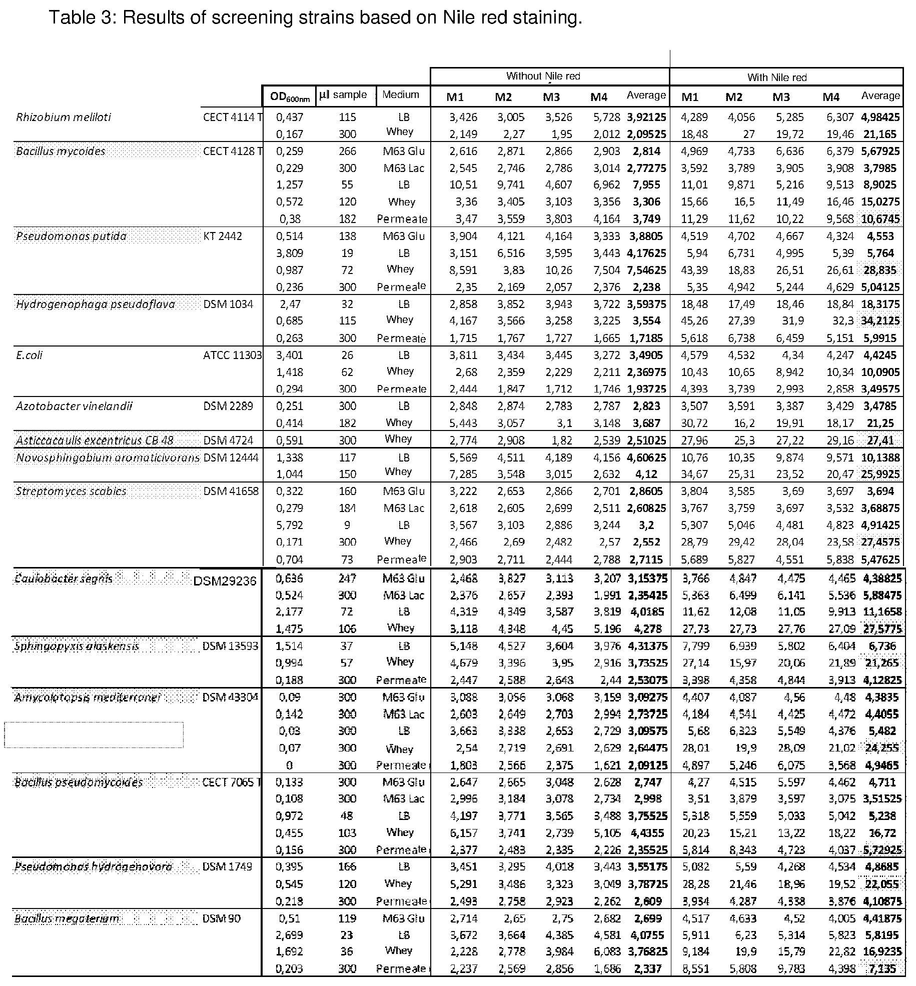

We selected four strains for further testing and quantification of PHA production: *A*. *mediterranei* DSM 43304, *C*. *segnis* DSM29236, *P. hydrogenovora* DSM 1749, and *B*. *megaterium* DSM 90.

These strains were precultured in MM-1 medium (Na₂SO₄ 0,5 g/L, KH₂PO₄ 7.5 g/L, (NH₄)₂HPO₄ 10 g/L, lactose 12 g/L, trace elements 25 mL/L (MgSO₄·7H2O 0,5 g/L, H₃BO₃ 2 mg/L, CuSO₄·5H₂O 1 mg/L, Na₂MoO₄·2H₂O 1,3 mg/L, ZnSO₄·7H₂O 11 mg/L, CoCl₂·6H₂O 2 mg/L, MnSO₄·H₂O 7 mg/L, FeSO₄·7H₂O 35 mg/L, KH₂PO₄ 2,5 g/L, CaCl₂·2H₂O 50 mg/L, citric acid 0.5 g/L, Kl 0.35 mg/L, Al₂(SO₄)₃ 0.5 g/L, with lactose as the sole carbon source in order to adapt to the presence of sugar.

Sugars in the culture medium (glucose and lactose) were analyzed by liquid chromatography in a Waters 1525/2695 with a differential refractive index detector and Rezex ROA Organic Acid column with H₂SO₄ at 2.5 mM and flow rate set at 0.5 mL/minute. Alternatively lactose concentration was determined using the enzymatic kit from Megazyme (K-LACGAR 12/11).

In tests with diluted whey, sugar consumption did not exceed 10%, therefore an optimization stage was included in conditions (paragraph d) supplementing whey with mineral salts.

### II. Selection of optimal fermentation process conditions for PHA production.

Different culture media were used to optimize PHA production with *C*. *segnis* DSM29236:
- Mineral medium 2 (MM2) (pH 7). Basal medium MA: Na₂HPO₄ 1.2 g/L, KH₂PO₄ 0.33 g/L, NH₄Cl 0.11 g/L, MgSO₄·7H₂O 0.1 g/L, CaCl₂ 0.04 g/L. Vitamins 1000x: biotin 20 mg/L, folic acid 20 mg / L, pyridoxine·HCl 10 mg/L, thiamine·HCl·2H₂O 50 mg/L, riboflavin 50 mg/L, niacin 50 mg/mL, D-pantothenate calcium 50 mg/L, vitamin B12 50 mg/L, p-aminobenzoic acid 50 mg/L. Trace elements 1000x: Nitrilotriacetic acid 1.5 g/L, ZnSO₄·7H₂O 0.18 g/L, MgSO₄·7H₂O 3 g/L, CuSO₄·5H₂O 0.01 g/L, MnCl₂·4H₂O 0.5 g/L, KAl(SO₄)₃·12H₂O 0.02 g/L, NaCl 1 g/L,H₃BO₃ 0.01 g/L, FeSO₄·7H₂O 0.1 g/L, Na₂MoO₄ 0.01 g/L, CoSO₄·7H₂O 0.18 g/L, NiCl₂·6H₂O 0.025 g/L, NaSeO₃·5H₂O 0.3 g/L.
- Mineral Medium 3 (MM3): (pH 7) Na₂HPO₄ 2.5 g/L, KH₂PO₄1.0 g/L. Trace elements 400x 5 M HCl: FeSO₄·7H₂O 10 g/L, CaCl₂·2H₂O 2 g/L, ZnSO₄·7H₂O 2.2 g/L, MnSO₄·4H₂O 0.5 g/L, CuSO₄·5H₂O 1 g/L, (NH₄)₆Mo₇O₂₄·4H₂O 0.1 g/L, Na₂B₄O₇·10H₂O 0.02 g/L, NiCl₂·6H₂O 8 mg/L, CoCl₂·6H₂O 2.4 mg/L.
- Mineral Medium 4 (MM4): whey supplemented with FeCl₃ and vitamins (pH 7).
- Mineral Medium 5 (MM5): Medium H3: NHCO₃0.5 g/L, Na₂HPO₄ 2.9 g/L, KH₂PO₄ 2.3 g/L, MgSO₄·7H₂O 0.5 g/L, (NH₄)₂SO₄ 2 g/L, CaCl₂·2H₂O 0.01 g/L, NH₄Fe(III) citrate 0.05 g/L, 5 mL of SL6 solution. Trace element solution SL6: ZnSO₄·7H₂O 100 mg/L, H₃BO₃ 300 mg/L, CoCl₂·6H₂O 200 mg/L, CuSO₄ 6 mg/L, NiCl₂·6H₂O 20 mg/L, Na₂MoO₄·12H₂O 30 mg/L, MnCl₂·2H₂O 25 mg/L.
- Mineral Medium 6 (MM6): Average MR: KH₂PO₄ 6.67 g/L, (NH₄)₂HPO₄ 4 g/L, MgSO₄·7H₂O 0.8 g/L, citric acid 0.8 g/L, 5 mL of solution trace elements. Trace element solution (5 M HCl): FeSO₄·7H₂O 10 g/L, CaCl₂·2H₂O 2 g/L, ZnSO₄·7H₂O 2.2 g/L, MnSO₄·4H₂O 0.5 g/L, CuSO₄·5H₂O 1 g/L, (NH₄)₆Mo₇O₂₄·4H₂O 0.1 g/L, Na₂B₄O₇·10H₂O 0.02 g/L.
- Mineral Medium 7 (MM7): (NH₄)₂SO₄ 5 g/L, Na₂HPO₄ 2.5 g/L, KH₂PO₄ 2.5 g/L, MgSO₄ 0.2 g/L, and MnSO₄ 0.01 g/L pH 7.

Whey was added to achieve an initial lactose concentration of 12 g/L. The bacteria were grown in flasks in incubators with stirring at 30 and 180 rpm. Growth was measured by absorbance at 600 nm in a spectrophotometer, or by counting colony forming units (CFU)/ml on agar plates.

PHA was determined by methanolysis and gas chromatography of samples of lyophilized culture. For this purpose, after 48 hours incubation, biomass was collected from the cultures by centrifugation and lyophilized. Methanolysis reaction was performed (H₂SO₄ in 15% in methanol and chloroform, methylbenzoate as an internal standard, for 4 hours at 100 °C) to transform the PHA into hydroxy methyl esters for analysis by gas chromatography. The resulting methyl esters were collected in phase with chloroform and analyzed by gas chromatography. M-toluic acid was used as internal standard and different pure commercial PHA were used as bioplastic standard: PHB (polyhydroxybutyrate), PHV (polyhydroxyvalerate), PHH (polyhydroxyhexanoate) and PHO (polyhydroxyalkanoate). The equipment used was an Agilent 6890N gas chromatograph with flame ionization detector (FID) and HP5-MS column.

The PHA produced by *C*. *segnis* DSM29236 was identified as PHB (poly-3-hydroxybutyrate) [Figure 1].

Sugars in the culture medium (glucose and lactose) were analyzed by liquid chromatography on a Waters 1525/2695 with differential refractive index detector and Rezex Organic Acid ROA column with H₂SO₄ at 2.5 mM, and 0.5 mL/minute flow rate.

Alternatively lactose concentration was determined using the enzymatic kit from Megazyme (K-LACGAR 12/11).

**Table 4. Growth data (OD₆₀₀) for PHB production and lactose consumption by C. segnis DSM29236.**

| **Medium** | **OD_{FinaL}** | **PHB (g/L)** | **Consumed lactose (g/L)** | **% Consumed lactose** |
|---|---|---|---|---|
| Dil. whey | n.d. | 0.009 | 1.47 | 12.2 |
| MM-2 | 1.53 | 0.071 | 3.22 | 31.1 |
| MM-3 | 1.92 | **0.093** | 1.25 | 10.5 |
| MM-4 | 1 | 0.019 | 3.41 | 35.2 |
| MM-5 | 1.29 | 0.036 | 2.31 | 20.5 |
| MM-6 | 1.4 | 0.036 | 2.24 | 18.8 |

The strain of *C*. *segnis* DSM29236 accumulated a little over 12%, reaching 0.09 g/L of PHB (Table 4) but lactose was not totally consumed. The highest PHB concentration was obtained with medium MM-3.

The process was optimized in micro-reactor tests (Applikon) in 24 wells of 3 mL. The temperature, pH and dissolved oxygen concentration were monitored and controlled via sensors in each well.

A factorial design of experiments based on the orthogonal matrix method, whereby representative results can be obtained using a number of chosen variables, fixed at different levels, with the minimum possible number of experiments (Table 5). This was intended to optimize both PHB production and lactose consumption starting with the simplest medium, MM-3, the medium for which the highest yields had been obtained. Therefore we decided to add nitrogen source and magnesium phosphates to the medium and also test variations in oxygen saturation.

**Table 5 Factors and levels considered in the experimental design**

| **Factors** | | **Levels** | | |
|---|---|---|---|---|
| **A** | (NH₄)₂SO₄ | 0.00 | 0.55 | 1.85 |
| **B** | MgSO₄ (g/L) | 0.0 | 0.4 | 0.8 |
| **C** | O₂ (%) | 10 | 20 | 30 |
| **D** | PO₄³⁻ (mM) | 10 | 20 | 30 |

Following the Taguchi method, experiments were designed with the L9 orthogonal array (Table 6) whereby nine experiments with 4 factors were run at 3 levels for each strain.

Further trials were included: i) a control experiment with the same conditions tested (MM-3 unchanged); ii) with added vitamins, and iii) with an intermediate nitrogen concentration (Table 7). Assays were carried out in the micro-reactor, with 3 mL of reaction volume, at 30 °C and stirring at 500 rpm. pH was left to develop without restraint. Results are shown in Table 8.

**Table 6. Matrix of the experimental design**

| **Exp.** | **Factors** | | | | **Variables** | | | |
|---|---|---|---|---|---|---|---|---|
| | **X_{A}** | **X_{B}** | **X_{C}** | **X_{D}** | **(NH₄)₂SO₄ (g/L)** | **MgSO₄ (g/L)** | **O₂(%)** | **PO₄³⁻ (mM)** |
| **1** | 1 | 1 | 1 | 1 | 0.00 | 0.0 | 10 | 10 |
| **2** | 1 | 2 | 2 | 2 | 0.00 | 0.4 | 20 | 20 |
| **3** | 1 | 3 | 3 | 3 | 0.00 | 0.8 | 30 | 30 |
| **4** | 2 | 1 | 1 | 3 | 0.55 | 0.0 | 10 | 30 |
| **5** | 2 | 2 | 3 | 1 | 0.55 | 0.4 | 30 | 10 |
| **6** | 2 | 3 | 2 | 2 | 0.55 | 0.8 | 20 | 20 |
| **7** | 3 | 1 | 3 | 2 | 1.85 | 0.0 | 30 | 20 |
| **8** | 3 | 2 | 1 | 3 | 1.85 | 0.4 | 10 | 30 |
| **9** | 3 | 3 | 2 | 1 | 1.85 | 0.8 | 20 | 10 |

**Table 7. Additional experiments**

| **Exp.** | **Medium MM-3** | **Variables** | | | |
|---|---|---|---|---|---|
| | **Modification** | **NH₄ (g/L)** | **MgSO₄ (g/L)** | **O₂ (%)** | **PO₄³⁻ (mM)** |
| **10** | Control | 0.0 | 0.0 | -- | 25 |
| **11** | Control + Vitamins | 0.0 | 0.0 | -- | 25 |
| **12** | Control + (NH₄)₂SO₄ | 1.0 | 0.0 | -- | 25 |

**Table 8. Optimization of the culture media**

| **Exp.** | **OD_{FinaL}** | **PHB (g/L)** | **Lactose (g/L)** | **%Lactose** | **Rto (%)** |
|---|---|---|---|---|---|
| **1** | 2.63 | 0.078 | 1.56 | 17.7 | 5.0 |
| **2** | 2.79 | 0.118 | 1.28 | 15.9 | 9.2 |
| **3** | 2.96 | 0.135 | 1.37 | 16.3 | 9.9 |
| **4** | 4.89 | 0.233 | 1.82 | 21.8 | 12.8 |
| **5** | 4.69 | 0.029 | 1.90 | 21.6 | 1.5 |
| **6** | 5.99 | 0.251 | 1.70 | 21.2 | 14.8 |
| **7** | 5.20 | 0.229 | - | - | - |
| **8** | 5.84 | 0.240 | 2.14 | 25.6 | 11.2 |
| **9** | 5.35 | 0.087 | 1.69 | 19.2 | 5.1 |
| **10** | 2.74 | 0.124 | 1.22 | 15.6 | 10.2 |
| **11** | 4.13 | 0.080 | n.d. | n.d. | - |
| **12** | 6.23 | 0.222 | n.d. | n.d. | - |

The concentration of PHA produced by *C*. *segnis* DSM29236 was increased, but lactose consumption rates were not entirely satisfactory in MM-3; therefore, subsequent optimization assays were performed in other culture media to try to increase sugar consumption.

*C*. *segnis* DSM29236 experiments were performed by modifying the inorganic nitrogen and the amount of whey in the culture media: MM-2, MM-3, MM-6 and MM-7. Table 9 shows the most relevant results. The conditions for these tests were:
(1) MM-6 with (NH₄)₂HPO₄ 3 g/L and KH₂PO₄ 6,67 g/L
(2) MM-6 with (NH₄)₂HPO₄3 g/L and without KH₂PO₄
(3) MM-6 with (NH₄)₂HPO₄ 1 g/L and KH₂PO₄ 6.67 g/L
(4) MM-6 with (NH₄)₂HPO₄ 4g/L and KH₂PO₄ 6.67 g/L
(5) MM-2 with 0.1 g/L (NH₄)₂SO₄ and 8 g/L lactose
(6) MM-2 with 0.1 g/L (NH₄)₂SO₄ and 16 g/L lactose
(7) MM-3 with 0.1 g/L (NH₄)₂SO₄ and 8 g/L lactose
(8) MM-3 with 0.2 g/L ((NH₄)₂SO₄ and 8 g/L lactose
(9) MM-3 without (NH₄)₂SO₄ and 8 g/L lactose
(10)MM-7 with 16 g/L lactose
(11)MM-7 with 8 g/L lactose

For the media MM-3, MM-2 and MM-7, the C/N ratio was modified so that combinations were made using half the amount of whey, twice the original amount of nitrogen and the original amounts thereof.

The best results were obtained with medium MM-3 under the conditions of experiment (7), in which 31.5% of PHB was achieved, and 1.50 g/L of PHB. Lactose consumption increased considerably compared to previous trials (Table 9).

**Table 9: Optimization of production of PHA with C.segnis DSM29236 with different culture media**

| **Exp.** | **OD_{Final}** | **PHB (%)** | **PHB (g/L)** | **% Lactose** |
|---|---|---|---|---|
| (1) | 2.41 | 7.4 | 0.15 | 23.6 |
| (2) | 3.83 | 12.2 | 0.38 | 36.7 |
| (3) | 3.31 | 9.2 | 0.25 | 42.6 |
| (4) | 2.49 | 7.4 | 0.15 | 32.3 |
| (5) | 2.99 | 5.9 | 0.14 | 70.8 |
| (6) | 5.51 | 8.5 | 0.45 | 63.4 |
| (7) | 5.89 | **31.5** | **1.50** | **74.5** |
| (8) | 5.52 | 23.1 | 1.04 | 66.5 |
| (9) | 4.05 | 22.4 | 0.78 | 65.0 |
| (10) | 5.02 | 7.7 | 0.43 | 52.7 |
| (11) | 2.69 | 8.8 | 0.20 | 80.9 |

### EXAMPLE 2. PHA production by C. segnis DSM29236 and C. crescentus from lactose contained in whey.

The optimum operating conditions were used with mineral culture medium (MM-3) supplemented with whey as a carbon source to produce PHA from lactose with *C*. *segnis* DSM29236.

Mineral medium 3 (MM3) contains: Na₂HPO₄ 2.5 g/L, KH₂PO₄ 1.0 g/L (pH 7). Trace elements 400x 5 M HCl: FeSO₄·7H₂O 10 g/L, CaCl₂·2H₂O 2 g/L, ZnSO₄·7H₂O 2.2 g/L, MnSO₄·4H₂O 0.5 g/L, CuSO₄·5H₂O 1 g/L, (NH₄)₆Mo₇O₂₄·4H₂O 0.1 g/L, Na₂B₄O₇·10H₂O 0.02 g/L, NiCl₂·6H₂O 8 mg/L, CoCl₂·6H₂O 2,4 mg/L. 0.1 g/L (NH₄)₂SO₄ and 8 g/L lactose.

Batch incubation was performed in a flask at 30°C and 180 rpm. The pH was set at 7 but changed freely during the course of fermentation.

Table 10 shows the results obtained from incubations at 48 hours.

**Table 10 PHA production by C. segnis DSM29236 and C. crescentus**

| | **OD_{Final}** | **PHB (%)** | **PHB (g/L)** | **% Lactose** |
|---|---|---|---|---|
| *C*. *segnis* DSM29236 | 5.89 | 31.5 | 1.50 | 74.5 |
| *C*. *crescentus* DSM | 1.05 | 11.1 | 0.07 | 25.1 |
| *C*. *crescentus* DSM | 1.15 | 2.4 | 0.02 | 27.9 |

Growth was monitored and measured by absorbance at 600 nm in a spectrophotometer, or by counting colony forming units (CFU)/ml on agar plates.

PHA was determined by methanolysis and gas chromatography of lyophilized samples of culture. To do so, after 48 hours of incubation, culture biomass was collected by centrifugation and lyophilized. A methanolysis reaction was performed (15% H₂SO₄ in methanol and chloroform, methylbenzoate as internal standard, for 4 hours at 100 °C) to transform the PHA into hydroxyacid methyl esters for subsequent analysis by gas chromatography. The resulting methyl esters were collected in phase with chloroform and analyzed by gas chromatography. Meta-toluic acid was used as an internal standard and the following pure commercial PHA bioplastics: PHB (polyhydroxybutyrate), PHV (polyhydroxyvalerate), PHH (polyhydroxyhexanoate) and PHO (polyhydroxyoctanoate). The apparatus used was an Agilent 6890N gas chromatograph with flame ionization detector (FID) and HP5-MS column. The PHA produced by *C*. *segnis* DSM29236 and *C*. *crescentus* was identified as PHB (poly-3-hydroxybutyrate).

The analysis of the sugars present in the culture medium (glucose and lactose) was performed by liquid chromatography using a Waters 1525/2695 equipment with differential refractive index detector and a Rezex Organic Acid ROA column with H₂SO₄ at 2.5 mM and 0.5 mL/ minute flow rate.

## Claims

1. A strain of the species *Caulobacter segnis* deposited at the German Collection of Microorganisms and Cell Cultures under deposit number DSM29236.

2. Strain according to claim 1, **characterized in that** it is in liquid, fresh paste, active dry or instant dry form.

3. Use of the strain according to claim 1 or 2 for the production of polyhydroxyalkanoates (PHA) from a solution comprising glucose, galactose and/or lactose.

4. Use according to claim 3, wherein the solution comprising glucose, galactose and/or lactose is whey or permeate resulting from the production of concentrated protein from whey.

5. Use according to claim 3 or 4, wherein the PHA is poly-3-hydroxybutyrate.

6. A method for producing PHA which comprises culturing a strain according to claim 1 or 2 in a culture medium under aerobic conditions, **characterized in that**:
- the culture medium comprises a source of phosphorus, a source of inorganic nitrogen and a solution comprising glucose, galactose and/or lactose, and
- the aerobic conditions comprise an oxygen saturation of at least 10%, preferably at least 20% or at least 30%.

7. Method according to claim 6, wherein the culture medium comprises between 2 and 20 g/L glucose, preferably, 4 g/L, between 2 and 20 g/L of galactose, preferably 4 g/L, and/or between 4 and 16 g/L of lactose, preferably, 8 g/L.

8. Method according to claim 6 or 7, wherein the culture medium comprises
(a) between 1.5 and 5.5 g/L of a phosphorus source, preferably 3.5 g/L and/or
(b) between 0.05 and 0.3 g/L of inorganic nitrogen source, preferably between 0.1 and 0.2 g/L.

9. Method according to any of claims 6 to 8, wherein the strain is cultured at a pH of between 7.5 and 6.5.

10. Method according to any of claims 6 to 9, wherein the cultivation of the strain is carried out at a temperature of between 28 and 32 °C.

11. Method according to any of claims 6 to 10, wherein the culture time is between 18 and 48 hours.

12. Method according to any of claims 6 to 11, wherein the solution comprising lactose, galactose and/or glucose is whey or permeate resulting from the production of concentrated protein from whey.

13. Method according to any of claims 6 to 12 which further comprises collecting the biomass obtained and extracting the PHA produced.

14. The method according to claim 13, wherein the PHA produced is extracted with an organic solvent.

15. Method according to any of claims 6 to 14, wherein the PHA is poly-3-hydroxybutyrate.

## Patentansprüche

1. Stamm der Art *Caulobacter segnis,* der bei der Deutschen Sammlung von Mikroorganismen und Zellkulturen unter der Hinterlegungsnummer DSM29236 hinterlegt ist.

2. Stamm nach Anspruch 1, **dadurch gekennzeichnet, dass** er in flüssiger Form, als frische Paste, aktiv trocknender oder sofort trocknender Form vorliegt.

3. Verwendung des Stammes nach Anspruch 1 oder 2 zur Herstellung von Polyhydroxyalkanoaten (PHA) aus einer Glukose, Galaktose und/oder Laktose umfassenden Lösung.

4. Verwendung nach Anspruch 3, wobei die Glukose, Galaktose und/oder Laktose umfassende Lösung Molke oder bei der Herstellung konzentrierter Proteine aus Molke entstehendes Permeat ist.

5. Verwendung nach Anspruch 3 oder 4, wobei das PHA Poly-3-hydroxybutyrat ist.

6. Verfahren zur Herstellung von PHA, das das Züchten eines Stammes nach Anspruch 1 oder 2 in einem Kulturmedium unter aeroben Bedingungen umfasst, **dadurch gekennzeichnet, dass**:
- das Kulturmedium eine Phosphorquelle, eine anorganische Stickstoffquelle und eine Glukose, Galaktose und/oder Laktose umfassende Lösung umfasst und
- die aeroben Bedingungen eine Sauerstoffsättigung von mindestens 10%, vorzugsweise mindestens 20% oder mindestens 30% umfassen.

7. Verfahren nach Anspruch 6, wobei das Kulturmedium zwischen 2 und 20 g/l, vorzugsweise 4 g/l Glukose, zwischen 2 und 20 g/l, vorzugsweise 4 g/l Galaktose und/oder zwischen 4 und 16 g/l, vorzugsweise 8 g/l Laktose umfasst.

8. Verfahren nach Anspruch 6 oder 7, wobei das Kulturmedium
(a) zwischen 1,5 und 5,5 g/l, vorzugsweise 3,5 g/l einer Phosphorquelle und/oder
(b) zwischen 0,05 und 0,3 g/l, vorzugsweise zwischen 0,1 und 0,2 g/l einer anorganischen Stickstoffquelle
umfasst.

9. Verfahren nach einem der Ansprüche 6 bis 8, wobei der Stamm bei einem pH zwischen 7,5 und 6,5 gezüchtet wird.

10. Verfahren nach einem der Ansprüche 6 bis 9, wobei die Züchtung des Stammes bei einer Temperatur zwischen 28 und 32°C durchgeführt wird.

11. Verfahren nach einem der Ansprüche 6 bis 10, wobei die Züchtungszeit zwischen 18 und 48 Stunden beträgt.

12. Verfahren nach einem der Ansprüche 6 bis 11, wobei die Laktose, Galaktose und/oder Glukose umfassende Lösung Molke oder bei der Herstellung konzentrierter Proteine aus Molke entstehendes Permeat ist.

13. Verfahren nach einem der Ansprüche 6 bis 12, das weiterhin das Sammeln der erhaltenen Biomasse und die Extraktion des hergestellten PHA umfasst.

14. Verfahren nach Anspruch 13, wobei das hergestellte PHA mit einem organischen Lösungsmittel extrahiert wird.

15. Verfahren nach einem der Ansprüche 6 bis 14, wobei das PHA Poly-3-hydroxybutyrat ist.

## Revendications

1. Une souche de l'espèce *Caulobacter segnis* déposée à la Collection Allemande de Microorganismes et de Cultures Cellulaires (DSMZ) sous le numéro de dépôt DSM29236.

2. Souche selon la revendication 1, **caractérisée en ce qu'**elle se trouve sous forme liquide, de pâte fraîche, desséchée active ou desséchée instantanée.

3. Utilisation de la souche selon la revendication 1 ou 2 pour la production de polyhydroxyalcanoates (PHA) à partir d'une solution comprenant du glucose, du galactose et/ou du lactose.

4. Utilisation selon la revendication 3, où la solution comprenant du glucose, du galactose et/ou du lactose est du lactosérum ou du perméat résultant de la production de protéine concentrée de lactosérum.

5. Utilisation selon la revendication 3 ou 4, où les PHA sont poly-3-hydroxybutyrate.

6. Un procédé pour produire des PHA qui consiste à cultiver une souche selon la revendication 1 ou 2 dans un milieu de culture dans des conditions d'aérobiose, **caractérisé en ce que** :
- le milieu de culture comprend une source de phosphore, une source d'azote inorganique et une solution comprenant du glucose, du galactose et/ou du lactose, et
- les conditions d'aérobiose comprennent une saturation en oxygène d'au moins 10%, de préférence d'au moins 20% ou d'au moins 30%.

7. Procédé selon la revendication 6, où le milieu de culture comprend entre 2 et 20 g/L de glucose, de préférence 4 g/L, entre 2 et 20 g/L de galactose, de préférence 4 g/L, et/ou entre 4 et 16 g/L de lactose, de préférence, 8 g/L.

8. Procédé selon la revendication 6 ou 7, où le milieu de culture comprend
(a) entre 1,5 et 5,5 g/L d'une source de phosphore, de préférence 3,5 g/L et/ou
(b) entre 0,05 et 0,3 g/L d'une source d'azote inorganique, de préférence entre 0,1 et 0,2 g/L.

9. Procédé selon n'importe laquelle des revendications 6 à 8, où la souche est cultivée à un pH compris entre 7,5 et 6,5.

10. Procédé selon n'importe laquelle des revendications 6 à 9, où la culture de la souche est réalisée à une température comprise entre 28 et 32°C.

11. Procédé selon n'importe laquelle des revendications 6 à 10, où le temps de culture est compris entre 18 et 48 heures.

12. Procédé selon n'importe laquelle des revendications 6 à 11, où la solution comprenant du lactose, du galactose et/ou du glucose est du lactosérum ou du perméat résultant de la production de protéine concentrée de lactosérum.

13. Procédé selon n'importe laquelle des revendications 6 à 12 qui comprend en outre le recueil de la biomasse obtenue et l'extraction des PHA produits.

14. Le procédé selon la revendication 13, où les PHA produits sont extraits avec un solvant organique.

15. Procédé selon n'importe laquelle des revendications 6 à 14, où les PHA sont poly-3-hydroxybutyrate.
